# Europäisches Patentamt
## European Patent Office
### Office européen des brevets

(19)

(11) Publication number: **0 153 780**
**B1**

(12)

# EUROPEAN PATENT SPECIFICATION

(45) Date of publication of patent specification: **05.04.89**

(51) Int. Cl.⁴: **C 07 C 1/04,** B 01 J 23/80, B 01 J 23/86

(21) Application number: **85200182.5**

(22) Date of filing: **12.02.85**

(54) Process for the preparation of hydrocarbons.

(30) Priority: **28.02.84 NL 8400609**

(43) Date of publication of application:
**04.09.85 Bulletin 85/36**

(45) Publication of the grant of the patent:
**05.04.89 Bulletin 89/14**

(84) Designated Contracting States:
**AT BE DE FR IT NL SE**

(56) References cited:
**EP-A-0 127 220**
**GB-A-2 077 289**
**GB-A-2 125 062**

(73) Proprietor: **SHELL INTERNATIONALE
RESEARCH MAATSCHAPPIJ B.V.
Carel van Bylandtlaan 30
NL-2596 HR Den Haag (NL)**

(72) Inventor: **Post, Martin Franciscus Maria
Badhuisweg 3
NL-1031 CM Amsterdam (NL)**
Inventor: **Sie, Swan Tiong
Badhuisweg 3
NL-1031 CM Amsterdam (NL)**

(74) Representative: **Aalbers, Onno et al
P.O. Box 302
NL-2501 CH The Hague (NL)**

EP 0 153 780 B1

# EP 0 153 780 B1

## Description

The invention relates to a process for the preparation of hydrocarbons by catalytic conversion of a mixture of carbon monoxide and hydrogen.

The preparation of hydrocarbons from a $H_2/CO$ mixture by contacting this mixture at elevated temperature and pressure with a catalyst is known in the literature as the Fischer-Tropsch hydrocarbon synthesis. Catalysts often used for the purpose comprise one or more metals from the iron group, together with one or more promotors, and a carrier material. These catalysts can suitably be prepared by the known techniques, such as precipitation, impregnation, kneading and melting. The products which can be prepared by using these catalysts generally have a very wide molecular weight distribution range and, in addition to branched and unbranched paraffins, they often contain considerable amounts of olefins and oxygen-containing organic compounds. Usually only a minor portion of the products obtained is made up of middle distillates. Of these middle distillates not only the yield but also the pour point is unsatisfactory. Therefore the direct conversion of $H_2/CO$ mixtures according to Fischer-Tropsch is not a very attractive route for the preparation of middle distillates on a technical scale.

In this patent application "middle distillates" should be taken to be hydrocarbon mixtures whose boiling range corresponds substantially with that of the kerosine and gas oil fractions obtained in the conventional atmospheric distillation of crude mineral oil. The middle distillate range lies substantially between about 150 and 360°C.

Recently there was found a class of Fischer-Tropsch catalysts having the property of yielding a product in which only very minor amounts of olefins and oxygen-containing compounds occur which consists virtually completely of unbranched paraffins, a considerable portion of which paraffins boils above the middle distillate range. It has been found that by hydrocracking the high-boiling part of this product can be converted in high yield into middle distillates. As feed for the hydrocracking at least the part of the product is chosen whose initial boiling point lies above the final boiling point of the heaviest middle distillate desired as end product. The hydrocracking, which is characterized by a very low hydrogen consumption, leads to middle distillates which have a considerably better pour point than those obtained in the direct conversion of a $H_2/CO$ mixture according to Fischer-Tropsch.

The Fischer-Tropsch catalysts belonging to the above-mentioned class contain silica, alumina or silica-alumina as carrier material and cobalt together with zirconium, titanium and/or chromium as catalytically active metals, in such quantities that per 100 pbw of carrier material, the catalysts comprise 3—60 pbw of cobalt and 0.1—100 pbw of zirconium, titanium or chromium. The catalysts are prepared by depositing the metals involved on the carrier material by kneading and/or impregnation. For further information on the preparation of these catalysts by kneading and/or impregnation reference may be made to Netherlands Patent Application No. 8301922 recently filed in the name of the Applicant.

When the present cobalt catalysts are used for the Fischer-Tropsch hydrocarbon synthesis starting from $H_2/CO$ mixtures having a $H_2/CO$ molar ratio of about 2, very high $H_2+CO$ conversions can be achieved. However, when feeds with lower $H_2/CO$ molar ratios are used, the $H_2+CO$ conversion is insufficient. The $H_2+CO$ conversion is seen to be lower according as the feed has a lower $H_2/CO$ molar ratio.

Since nature provides large amounts of material with a relatively low H/C ratio such as coal, which when converted into $H_2/CO$ mixtures yields products having $H_2/CO$ molar ratios lower than 2, it would naturally be very welcome if a way could be found to solve the afore-mentioned problem of low $H_2+CO$ conversions.

In British patent application No. 2,077,209 the use of $H_2/CO$ mixtures having an $H_2/CO$ ratio less than 1.5 in a process for the preparation of hydrocarbons has been described. In this process water is added to the feed whereafter the feed is first contacted with a CO-shift catalyst followed by contacting the thus obtained reaction mixture with a cobalt catalyst. The $C_5^+$-selectivity of the process, however, is not yet satisfactory.

During an investigation of the use of $H_2/CO$ mixtures having a low $H_2/CO$ ratio two measures were found which have made it possible to realise high $H_2+CO$ conversions in the hydrocarbon synthesis starting from $H_2/CO$ mixtures having $H_2/CO$ molar ratios between 0.25 and 1.75 and by using the present cobalt catalysts. In addition the application of these measures leads to a high $C_5^+$ selectivity. By the first measure the $H_2/CO$ mixture is converted over a mixture of two catalysts, one of which is the cobalt catalyst and the other a copper- and zinc-containing composition. By the second measure the $H_2/CO$ mixture is first partly converted over the cobalt catalyst, and subsequently the unconverted $H_2$ and CO is converted over a bifunctional catalyst or catalyst combination which, in addition to activity for the conversion of a $H_2/CO$ mixture into hydrocarbons, has activity for the conversion of a mixture of $H_2O$ and CO into a mixture of $H_2$ and $CO_2$.

Conditional upon the $H_2/CO$ molar ratio of the feed to be converted it is either exclusively measure 1, or exclusively measure 2, or either one of the two measures that is eligible for use. For feeds with a $H_2/CO$ molar ratio between 0.25 and 0.75 only measure 1 is applicable. If the feed has a $H_2/CO$ molar ratio between 1.0 and 1.75, it is only measure 2 that is eligible. For feeds with a $H_2/CO$ molar ratio between 0.75 and 1.0 either measure 1 or measure 2 can be used at choice.

The present patent application relates to the use of measure 1 for feeds with a $H_2/CO$ molar ratio between 0.25 and 1.0. The use of measure 2 for feeds having a $H_2/CO$ molar ratio between 0.75 and 1.75 forms the subject matter of European Patent Application 153,781.

2

In the catalyst mixture which according to measure 1 is used for the conversion of the $H_2$/CO mixture the copper- and zinc-containing composition should have a Cu/Zn atomic ratio in the range between 0.1 and 10. In addition the ratio in which the two catalysts are present in the catalyst mixture should be such as to satisfy the relation

$$0,5 \times \frac{2-F}{1+F} < M < 5 \times \frac{2-F}{1+F},$$

wherein F represents the $H_2O$/CO molar ratio of the feed and M the (Cu+Zn)/Co atomic ratio in the catalyst mixture.

The present patent application therefore relates to a process for the preparation of hydrocarbons by catalytic reaction of a mixture of carbon monoxide and hydrogen with an $H_2$/CO molar ratio of less than 1.0, in which the mixture is contacted at elevated temperature and pressure with two catalysts, one catalyst being a catalyst comprising cobalt, at least one metal chosen from the group formed by zirconium, titanium and chromium with a carrier, and the other catalyst being a copper- and zinc-containing composition, characterised in that the $H_2$- and CO-containing feed has a $H_2$/CO molar ratio (F) in the range between 0.25 and 1.0, the one catalyst comprising 3—60 pbw of cobalt and 0.1—100 pbw of the metal chosen from the group formed by zirconium, titanium and chromium per 100 pbw of the carrier chosen from silica, alumina or silica-alumina and which has been prepared by kneading and/or impregnation, and the other catalyst having a Cu/Zn atomic ratio in the range between 0.1 and 10, and that the two catalysts are present in the catalyst mixture in such a ratio as to satisfy the relation

$$0.5 \times \frac{2-F}{1+F} < M < 5 \times \frac{2-F}{1+F},$$

wherein M represents the (Cu+Zn)/Co atomic ratio in the catalyst mixture.

In the process of the invention it is preferred to use the cobalt catalysts which form the subject matter of Netherlands patent application No. 8301922. These are catalysts which satisfy the relation

$$(3+4 \ R) > \frac{L}{S} > (0.3+0.4 \ R),$$

wherein

L=the total quantity of cobalt present on the catalyst, expressed as mg Co/ml catalyst,

S=the surface area of the catalyst, expressed as $m^2$/ml catalyst, and

R=the weight ratio of the quantity of cobalt deposited on the catalyst by kneading, to the total quantity of cobalt present on the catalyst.

The preparation of the cobalt catalysts which are used according to the invention is preferably carried out according to one of the three procedures mentioned hereinafter:

a) first cobalt is deposited in one or more steps by impregnation and subsequently the other metal is deposited in one or more steps, also by impregnation,

b) first the other metal is deposited in one or more steps by impregnation and subsequently the cobalt is deposited in one or more steps, also by impregnation, and

c) first cobalt is deposited in one or more steps by kneading and subsequently the other metal is deposited in one or more steps by impregnation.

In the process according to the invention preference is given to the use of cobalt catalysts containing 15—50 pbw of cobalt per 100 pbw of carrier. The preferred quantity of other metal present in the cobalt catalysts depends on the way in which this metal has been deposited. In the case of catalysts where first cobalt has been deposited on the carrier, followed by the other metal, preference is given to catalysts containing 0.1—5 pbw of the other metal per 100 pbw of carrier. In the case of catalysts where first the other metal has been deposited on the carrier, followed by the cobalt, preference is given to catalysts containing 5—40 pbw of the other metal per 100 pbw of carrier. Preference is given to zirconium as other metal and to silica as carrier material.

The copper- and zinc-containing composition which in the process according to the invention is used as a component of the catalyst mixture preferably has a Cu/Zn atomic ratio in the range between 0.25 and 4. Preparatory to being suitable for use the catalyst mixtures should be activated. This activation can suitably be carried out by contacting the catalyst mixture with hydrogen or a hydrogen-containing gas, first at a temperature between 150 and 250°C and subsequently at a higher temperature, between 200 and 350°C.

The process according to the invention is preferably carried out at a temperature of 125—350°C and a pressure of 5—100 bar. Special preference is given to a temperature of 175—275°C and a pressure of 10—75 bar.

As explained hereinbefore, the present cobalt catalysts when used for the conversion of a $H_2$- and

CO-containing feed yield a product which is substantially waxy and whose high-boiling part can be converted in high yield into middle distillates by using a hydrocracking treatment. This is also true when, instead of the cobalt catalysts alone, the catalyst mixtures proposed here are used.

Although in the preparation of middle distillates from the product obtained over the catalyst mixture the part of the product whose initial boiling point lies above the final boiling point of the heaviest middle distillate desired as end product will do as feed for the hydrocracking, it is preferred to use for this purpose the total $C_5^+$ fraction of the product prepared over the catalyst mixture, since it has been found that the catalytic hydrotreatment leads to enhanced quality of the gasoline, kerosine and gas oil fractions present therein.

The hydrocracking is carried out by contacting the fraction to be treated at elevated temperature and pressure and in the presence of hydrogen with a catalyst containing one or more noble metals from Group VIII supported on a carrier. The hydrocracking catalyst used by preference is a catalyst containing 0.1—2 %w and in particular 0.2—1%w of one or more noble metals from Group VIII supported on a carrier. Preference is given to catalysts comprising platinum or palladium as Group VIII noble metal and silica-alumina as carrier. The hydrocracking is preferably carried out at a temperature of 200—400°C and in particular of 250—350°C and a pressure of 5—100 bar and in particular of 10—75 bar.

The invention is now illustrated with the aid of the following example.

## Example

Starting from three catalysts (1—3) five catalyst mixtures (I—V) were composed by mixing.

## Catalyst 1

Co/Zr/SiO$_2$ catalyst which comprised 25 pbw of cobalt and 0.9 pbw of zirconium per 100 pbw of silica and had been prepared by single-step impregnation of a silica carrier with a solution of cobalt nitrate in water, followed by single-step impregnation of the cobalt-loaded carrier with a solution of zirconium nitrate in water. The catalyst had a value for L of 98 mg/ml and for S of 96 m$^2$/ml and therefore for L/S of 1.02 mg/m$^2$.

## Catalyst 2

Co/Zr/SiO$_2$ catalyst which comprised 25 pbw of cobalt and 12 pbw of zirconium per 100 pbw of silica and had been prepared by three-step impregnation of a silica carrier with a solution fo zirconium tetra n-propoxide in a mixture of n-propanol and benzene, followed by impregnation of the zirconium-loaded carrier with a solution of cobalt nitrate in water. The catalyst had a value for L of 97 mg/ml and for S of 100 m$^2$/ml and therefore for L/S of 0.97 mg/m$^2$.

In the preparation of Catalysts 1 and 2 a quantity of solution was used in each impregnation step which corresponded substantially with the pore volume of the carrier and the material was dried after each impregnation step and then calcined at 500°C.

## Catalyst 3

Cu/Zn/Al$_2$O$_3$ catalyst which comprised 24.3 %w of copper and 38.0 %w of zinc and therefore had a Cu/Zn atomic ratio of 0.66.

Catalyst 3 was mixed with Catalyst 1 or Catalyst 2 to prepare the catalyst mixtures I—V having (Cu+Zn)/Co atomic ratios (M) ranging from 0.19 to 21.8. The mixing components of which each catalyst mixture was composed as well as the value for M of each catalyst mixture are given in Table I.

## Catalyst testing

Catalyst mixtures I—V were used in eight experiments (1—8) in the preparation of hydrocarbons from mixtures of carbon monoxide and hydrogen. The experiments were carried out at a temperature of 250°C, a pressure of 20 bar and a space velocity of 400 Nl gas/l catalyst mixture/h in a reactor containing a fixed catalyst bed. Before they were subjected to the testing the catalyst mixtures were activated by contacting them with a hydrogen-containing gas, first at 200°C and then at 250°C. The results of the experiments and the H$_2$/Co molar ratios (F) of the feeds used in each one of the experiments are given in Table II.

The parameters H$_2$+CO conversion and C$_5^+$ selectivity mentioned in Table II are defined as follows:

$$H_2+CO \ \text{Conversion} = \frac{\text{mol } H_2+CO \text{ in feed} - \text{mol } H_2+CO \text{ in product}}{\text{mol } H_2+CO \text{ in feed}} \times 100,$$

$$C_5^+ \ \text{Selectivity} = \frac{\text{pbw } C_5^+ \text{ hydrocarbons in product}}{\text{pbw hydrocarbons in product}} \times 100.$$

Of the experiments mentioned in Table II Experiments 3, 5 and 6 are experiments according to the invention. These experiments, in which the relation according to the invention of M of the catalyst mixture to F of the feed was satisfied, yielded both high conversions and high C$_5^+$ selectivities. Experiments 1, 2, 4,

# EP 0 153 780 B1

7 and 8 fall outside the scope of the invention. They have been included in the patent application for comparison. These experiments, in which the relation according to the invention of M of the catalyst mixture and F of the feed was not satisfied, yielded too low conversions and/or too low $C_5^+$ selectivities.

TABLE I

| Catalyst mixture | Catalyst mixing components | M |
|---|---|---|
| I | 1+3 | 21,8 |
| II | 1+3 | 3,75 |
| III | 2+3 | 1,41 |
| IV | 1+3 | 0,85 |
| V | 1+3 | 0,19 |

TABLE II

| Experiment No. | Catalyst mixture No. | F | $H_2+CO$ conversion, %mol | $C_5^+$ selectivity, %w |
|---|---|---|---|---|
| 1 | I | 0,55 | 45 | 75 |
| 2 | I | 0,90 | 65 | 68 |
| 3 | II | 0,55 | 84 | 85 |
| 4 | II | 0,90 | 82 | 70 |
| 5 | III | 0,55 | 91 | 86 |
| 6 | IV | 0,90 | 91 | 77 |
| 7 | V | 0,55 | 54 | 87 |
| 8 | V | 0,90 | 74 | 86 |

## Claims

1. A process for the preparation of hydrocarbons by catalytic reaction of a mixture of carbon monoxide and hydrogen with an $H_2/CO$ molar ratio of less than 1.0, in which the mixture is contacted at elevated temperature and pressure with two catalysts, one catalyst being a catalyst comprising cobalt, at least one metal chosen from the group formed by zirconium, titanium and chromium and a carrier, and the other catalyst being a copper- and zinc-containing composition, characterised in that the $H_2$- and CO-containing feed has a $H_2/CO$ molar ratio (F) in the range between 0.25 an 1.0, the one catalyst comprising 3—60 pbw of cobalt and 0.1—100 pbw of the metal chosen from the group formed by zirconium, titanium and chromium per 100 pbw of th carrier chosen from silica, alumina or silica-alumina and which has been prepared by kneading and/or impregnation, and the other catalyst having a Cu/Zn atomic ratio in the range between 0.1 and 10, and that the two catalysts are present in the catalyst mixture in such a ratio as to satisfy the relation

$$0.5\times\frac{2-F}{1+F} <M<5\times \frac{2-F}{1+F},$$

wherein M represents the (Cu+Zn)/Co atomic ratio in the catalyst mixture.

2. A process as claimed in claim 1, characterized in that the cobalt catalyst satisfies the relation

$$(3+4\ R)>\frac{L}{S}>(0.3+0.4\ R),$$

wherein

5

L=the total quantity of cobalt present on the catalyst, expressed as mg Co/ml catalyst,
S=the surface area of the catalyst, expressed as $m^2$/ml catalyst, and
R=the weight ratio of the quantity of cobalt deposited on the carrier by kneading, to the total quantity of coablt present on the catalyst.

3. A process as claimed in claim 1 or 2, characterized in that per 100 pbw of carrier the cobalt catalyst comprises 15—50 pbw of cobalt and either 0.1—5 pbw of the other metal if during the preparation cobalt was deposited first and the other metal next, or 5—40 pbw of the other metal if during the preparation the other metal was deposited first and cobalt next.

4. A process as claimed in any one of claims 1—3, characterized in that the cobalt catalyst contains zirconium as other metal and silica as carrier.

5. A process as claimed in any one of claims 1—4, characterized in that the copper- and zinc-containing composition has a Cu/Zn atomic ratio in the range between 0.25 and 4.

6. A process as claimed in any one of claims 1—5, characterized in that it is carried out at a temperature of 125—350°C and a pressure of 5—100 bar.

7. A process as claimed in claim 6, characterized in that it is carried out at a temperature of 175—275°C and a pressure of 10—75 bar.

8. A process as claimed in any one of claims 1—7, characterized in that in order to prepare middle distillates from the product obtained over the catalyst mixture, at least the part whose initial boiling point lies above the final boiling point of the heaviest middle distillate desired as end product, is subjected to hydrocracking by contacting it at elevated temperature and pressure with a catalyst comprising one or more noble metals from Group VIII supported on a carrier.

## Patentansprüche

1. Verfahren zur Herstellung von Kohlenwasserstoffen durch katalytische Umsetzung eines Gemisches aus Kohlenmonoxid und Wasserstoff mit einem $H_2$/CO-Molverhältnis von weniger als 1,0, in welchem Verfahren das Gemisch bei erhöhter Temperatur und bei erhöhtem Druck mit 2 Katalysatoren in Berührung gebracht wird, wobei der eine Katalysator ein Kobalt, wengistens ein Metall, ausgewählt aus der aus Zirkon, Titan und Chrom gebildeten Gruppe, und einen Träger umfassender Katalysator ist und der andere Katalysator eine Kupfer und Zink enthaltende Zusammensetzung ist, dadurch gekennzeichnet, daß das $H_2$ und CO enthaltende Einsatzmaterial ein $H_2$/CO-Molverhältnis (F) im Bereich von 0,25 bis 1,0 aufweist, der eine Katalysator 3 bis 60 Gewichtsteile Kobalt und 0,1 bis 100 Gewichtsteile des Metalles, ausgewählt aus der aus Zirkon, Titan und Chrom gebildeten Gruppe, je 100 Gewichtsteile des unter Siliziumdioxid, Aluminiumoxid oder Siliziumdioxid-Aluminiumoxid ausgewählten Trägers umfaßt und der durch Kneten und/oder Imprägnieren hergestellt worden ist, und der andere Katalysator ein Cu/Zn-Atomverhältnis im Bereich von 0,1 bis 10 aufweist, und daß die beiden Katalysatoren in dem Katalysatorgemisch in einem solchen Verhältnis vorliegen, daß der Beziehung

$$0{,}5 \times \frac{2-F}{1+F} < M < 5 \times \frac{2-F}{1+F}$$

entsprochen wird, worin M das (Cu+Zn)/Co-Atomverhältnis im Katalysatorgemisch darstellt.

2. Verfahren nach Anspruch 1, dadurch gekennzeichnet, daß der Kobaltkatalysator der Beziehung

$$(3+4 \text{ R}) > \frac{L}{S} > (0{,}3+0{,}4 \text{ R})$$

entspricht, worin
L=Gesamtmenge an auf dem Katalysator vorliegendem Kobalt, ausgedrückt als mg Co/ml Katalysator,
S=Oberfläche des Katalysators, ausgedrückt als $m^2$/ml des Katalysators, und
R=Gewichtsverhältnis der auf dem Katalysator durch Kneten abgelagerten Kobaltmenge zu der auf dem Katalysator vorliegenden Gesamtmenge an Kobalt.

3. Verfahren nach Anspruch 1 oder 2, dadurch gekennzeichnet, daß je 100 Gewichtsteile Träger der Kobaltkatalysator 15 bis 50 Gewichtsteile Kobalt und entweder 0,1 bis 5 Gewichtsteile des anderen Metalles enthält, wenn im Zuge der Herstellung Kobalt als erste und das andere Metall als nächstes abgelagert worden sind, oder 5 bis 40 Gewichtsteile des anderen Metalles enthält, wenn im Zuge der Herstellung das andere Metall als erstes und hierauf Kobalt abgelagert worden ist.

4. Verfahren nach einem der Ansprüche 1 bis 3, dadurch gekennzeichnet, daß der Kobaltkatalysator Zirkon als anderes Metall und Siliziumdioxid als Träger enthält.

5. Verfahren nach einem der Ansprüche 1 bis 4, dadurch gekennzeichnet, daß die Kupfer und Zink enthaltende Zusammensetzung ein Cu/Zn-Atomverhältnis im Bereich von 0,25 bis 4 aufweist.

6. Verfahren nach einem der Ansprüche 1 bis 5, dadurch gekennzeichnet, daß es bei einer Temperatur von 125 bis 350°C und bei einem Druck von 5 bis 100 bar ausgeführt wird.

7. Verfahren nach Anspruch 6, dadurch gekennzeichnet, daß es bei einer Temperatur von 175 bis 275°C und bei einem Druck von 10 bis 75 bar ausgeführt wird.

8. Verfahren nach einem der Ansprüche 1 bis 7, dadurch gekennzeichnet, daß zur Herstellung von Mitteldestillaten aus dem über dem Katalysatorgemisch erhaltenen Produkt zumindest jener Teil des Produktes, dessen Anfangssiedepunkt oberhalb des Endsiedepunktes des als Endprodukt gewünschten schwersten Mitteldestillates liegt, einem Hydrocracken unterzogen wird, indem er bei erhöhter Temperatur und bei erhöhtem Druck mit einem Katalysator in Berührung gebracht wird, der ein oder mehrere Edelmetalle aus der Gruppe VIII, aufgebracht auf einem Träger, umfaßt.

**Revendications**

1. Procédé de préparation d'hydrocarbures par réaction catalytique d'un mélange de monoxyde de carbone et d'hydrogène, de rapport molaire $H_2/CO$ inférieur à 1,0, dans lequel le mélange est mis en contact, à une température élevée et sous une pression élevée, avec deux catalyseurs, l'un des catalyseurs étant un catalyseur comprenant du cobalt, au moins un métal choisi dans le groupe formé par le zirconium, le titane et le chrome, et un véhicule, et l'autre catalyseur étant une composition contenant du cuivre et du zinc, caractérisé en ce que l'alimentation contenant $H_2$ et CO a un rapport molaire $H_2/CO$ (F) dans l'intervalle compris entre 0,25 et 1,0, le premier catalyseur comprenant 3—60 parties en poids de cobalt et 0,1—100 parties en poids du métal choisi dans le groupe formé par le zirconium, le titane et le chrome, pour 100 parties en poids du support choisi parmi la silice, l'alumine ou la silice-alumine et ayant été préparé par pétrissage et/ou imprégnation, et l'autre catalyseur ayant un rapport atomique Cu/Zn dans l'intervalle compris entre 0,1 et 10, et en ce que les deux catalyseurs sont présents dans le mélange catalytique en un rapport tel qu'il satisfasse à la relation

$$0,5 \times \frac{2-F}{1+F} < M < 5 \times \frac{2-F}{1+F},$$

dans laquelle M représente le rapport atomique (Cu+Zn)/Co dans le mélange catalytique.

2. Procédé selon la revendication 1, caractérisé en ce que le catalyseur au cobalt satisfait à la relation

$$(3+4\ R) > \frac{L}{S} > (0,3+0,4\ R),$$

dans laquelle

L = quantité totale de cobalt présente sur le catalyseur, exprimée en mg de Co/ml de catalyseur,
S = surface spécifique du catalyseur, exprimée en $m^2$/ml de catalyseur, et
R = rapport pondéral de la quantité de cobalt déposée sur le catalyseur par pétrissage à la quantité totale de cobalt présente sur le catalyseur.

3. Procédé selon la revendication 1 ou 2, caractérisé en ce que, pour 100 parties en poids de support, le catalyseur au cobalt comprend 15—50 parties en poids de cobalt et ou bien 0,1—5 parties en poids de l'autre métal si, au cours de la préparation, le cobalt a été déposé en premier et l'autre métal ensuite, ou bien 5—40 parties en poids de l'autre métal si, au cours de la préparation, l'autre métal a été déposé en premier et le cobalt ensuite.

4. Procédé selon l'une quelconque des revendications 1—3, caractérisé en ce que le catalyseur au cobalt contient du zirconium comme autre métal et de la silice comme support.

5. Procédé selon l'une quelconque des revendications 1—4, caractérisé en ce que la composition contenant du cuivre et du zinc a un rapport atomique Cu/Zn dans l'intervalle compris entre 0,25 et 4.

6. Procédé selon l'une quelconque des revendications 1—5, caractérisé en ce qu'il est mis en oeuvre à une température de 125—350°C et sous une pression de 5—100 bars.

7. Procédé selon la revendication 6, caractérisé en ce qu'il est mis en oeuvre à une température de 175—275°C et sous une pression de 10—75 bars.

8. Procédé selon l'une quelconque des revendications 1—7, caractérisé en ce que, afin de préparer des distillats moyens à partir du produit obtenu sur le mélange catalytique, on soumet à un hydrocraquage au moins la partie dont le point d'ébullition initial se situe au-dessus du point d'ébullition final du distillat moyen le plus lourd que l'on souhaite comme produit final en la mettant en contact, à une température élevée et sous une pression élevée, avec un catalyseur comprenant un ou plusieurs métaux nobles du groupe VIII supportés sur un support.